# EUROPÄISCHE PATENTSCHRIFT

(11) **EP 1 491 294 B1**
(45) Veröffentlichungstag und Bekanntmachung des Hinweises auf die Patenterteilung: **18.10.2006**
(21) Anmeldenummer: 03013952.1
(22) Anmeldetag: 20.06.2003
(51) Int. Cl.: B25B 23/142, A61B 17/88

(54) **Eindrehwerkzeug für Implantatschrauben**
Tool for the insertion of implant screws
Outil pour l'insertion des implants à vis

(43) Veröffentlichungstag der Anmeldung: 29.12.2004
(73) Patentinhaber: Zimmer GmbH, 8404 Winterthur (CH)
(72) Erfinder: Casutt, Simon, 9200 Gossau (CH)
(74) Vertreter: Manitz, Finsterwald & Partner GbR

(56) Entgegenhaltungen:
- EP-A- 1 234 637
- WO-A-00/38589
- DE-A- 4 200 364
- FR-A- 1 386 414
- US-A- 2 151 953
- US-A- 2 256 478
- US-A- 2 461 491
- US-A- 3 783 682
- US-A- 6 132 435
- US-B1- 6 575 042

## Beschreibung

Die Erfindung handelt von einem Eindrehwerkzeug für Implantatschrauben mit einem Griff und mit einem stabförmigen Werkzeug gemäß dem Oberbegriff des Anspruchs 1. Ein derartiges Werkzeug ist aus US 2,256,478 bekannt.
Implantatschrauben einer bestimmten Grösse dürfen in der Regel nur mit einem begrenzten Drehmoment zur Verbindung von Implantatteilen angezogen werden, damit das Gewinde oder die zusammengepressten Implantatteile keinen Schaden nehmen. Andererseits muss eine Implantatschraube mit einem Mindestmoment angezogen werden, damit sie sich nicht löst. Der Hersteller von verschraubbaren Implantaten sollte daher die Möglichkeit geben eine solche Verschraubung unter Einhaltung eines vorgegebenen Drehmoments vorzunehmen. Werkzeuge zum Eindrehen solcher Schrauben sollten wegen ihrer mehrfachen Verwendung gut sterilisierbar sein und keinen Abrieb aufweisen. In der US Patentschrift Nr. 5,048,381 ist ein für nicht sterile Bereiche zulässiges Eindrehwerkzeug mit einer Drehmomentmessung aufgezeigt. Die US Patentschrift 5,737,983 zeigt ebenfalls allgemeine Eindrehwerkzeuge mit einem Torsionsstab, welcher in seiner Bewegung gegen Überbelastung begrenzt ist, wobei diese Grenze taktil als Widerstand spürbar ist, um mit Sicherheit ein Mindestmoment erreicht zu haben. Im Gegensatz zu den technischen Verschraubungen, bei denen ein Mindetsanzugsmoment gegen das Lösen der Verschraubung gefordert wird, darf bei einer Implantatschraube ein bestimmtes Anzugsmoment nicht überschritten werden. Eine in ihrer Erhöhung nicht kontrollierte Überbeanspruchung ist daher als Grenze für das Eindrehen einer Implantatschraube gleich wie das subjektive Gefühl beim Eindrehen mit einem normalen Schraubendreher. Aufgabe der Erfindung ist es, dem operierenden Arzt das Erreichen eines zulässigen Eindrehmomentes mit Sicherheit kenntlich zu machen, wobei gleichzeitig das Eindrehwerkzeug bei einfachem Aufbau möglichst stabil ausgeführt sein soll. Diese Aufgabe wird erfindungsgemäß gelöst durch die Merkmale des Anspruchs 1. Ein derartiges Werkzeug hat den Vorteil, dass im Gegensatz zu einem auf den Griff beschränkten Torsionsstab ein grösserer Anzeigewinkel für ein aufgebrachtes Drehmoment möglich ist. Eine schraubenförmige hohle Wendel stellt bei einem gleich grossen Drehmoment einen wesentlichen grösseren Energiespeicher dar d.h. es entsteht ein grösserer Drehwinkel bei kleinerer spezifischer Belastung, wobei die hohle Wendel auch noch in einem relativ kleinen und für den Handgriff zweckmässigen Raum untergebracht ist. Im Grunde genommen entsteht eine weichere Torsionsfeder, die aber ohne zu grosse lokale Spannungsspitzen über einen grossen Winkelbereich betätigbar ist. Man erkennt erst durch eine theoretische Abwicklung der schraubenförmigen Spirale, dass hier vielmehr Länge für eine elastische Biegeverformung zur Verfügung steht, wobei in jedem Querschnitt das gleiche Biegemoment auftritt. Die abhängigen Ansprüche stellen vorteilhafte Weiterbildungen der Erfindung dar. Die Drehrichtung der Wendel entspricht der Drehrichtung des Schraubengewindes, wenn eine Implantatschraube eingedreht wird. Beim Eindrehen wird die Wendel daher ihren Durchmesser verringern und auch in axialer Richtung Kräfte ausüben. Aus diesem Grund ist es zweckmässig den in Drehrichtung beweglichen Kopfteil mit einer in beiden Richtungen wirksamen Axialverriegelung relativ zum Längsgriff zu versehen. Eine solche Axialverriegelung verhindert auch ein Zurückfedern beim Ansetzen des Eindrehwerkzeuges an eine Implantatschraube.
Mit einem Anschlag im Griffteil, kann eine Drehung des Kopfteils, die weit über dem mit einem Zeiger angezeigbaren vorgesehenen Drehmoment liegt, begrenzt werden und so einer plastischen Verformung der hohlen Wendel vorgebeugt werden. Die Begrenzung kann an einem mit dem Kopfteil verbundenen Bolzen oder Schraube erfolgen, welche in einem Schlitz des Griffteils laufen. Ein Schlitz mit einem Drehwinkel zwischen 30° und 90° ergibt einen für die Praxis vernünftigen Anzeigebereich für ein zulässiges Drehmoment und dieser Winkel lässt sich ohne Umsetzen mit einer Hand am Griff erzeugen. Wenn der Werkzeugteil mit dem Kopfteil lösbar gekoppelt ist, lassen sich verschiedene Werkzeugteile und Griffe mit verschieden grosser Drehmomentanzeige in einem Baukasten miteinander kombinieren. Wenn der Längsgriff aus einem sterilisierbaren Kunststoff besteht, wie er bei Instrumentenhaltern üblich ist, und wenn die restlichen Teile aus Metall, beispielsweise aus nicht rostenden Metallegierungen bestehen, ist eine Sterilisation im Ganzen möglich. Zur symmetrischen Kraftverteilung kann die Wendel zweigängig als Doppelwendel ausgeführt sein. Kopfteil, Wendel und Fussteil sind erfindungsgemäß einstückig ausgeführt, womit weitere Fügestellen und potentielle Fehlerquellen vermieden werden.
Wenn, wie oben beschrieben, ein relativ grosser Winkel zur Ablesung des Drehmomentes zur Verfügung steht, ist nicht nur die Ablesung recht genau sondern es kann auch an der Null-Lage ein Toleranzfeld für die Null-Lage angebracht werden, an welchem man erkennt, ob sich die Null-Lage beispielsweise wegen Überbeanspruchung im ungesicherten Zustand d.h. ohne Inanspruchnahme des oben aufgeführten Anschlags verschoben hat. Je grösser der Anzeigewinkel für ein zulässiges Drehmoment ist, desto grösser kann auch der Toleranzbereich für die Null-Lage gewählt werden. Bei einem Drehwinkel von 60° kann beispielsweise die Null-Lage eine Toleranz von plus/minus 2° aufweisen, was einem noch gut sichtbaren Kontrollbereich entspricht.

Im folgenden wird die Erfindung anhand von Ausführungsbeispielen beschrieben. Es zeigen:
- Figur 1:: schematisch ein erfindungsgemässes Eindrehwerkzeug im Längsschnitt;
- Figur 2:: schematisch eine Seitenansicht des Werkzeuges in Figur 1;
- Figur 3:: schematisch eine Draufsicht auf den rückwärtigen Teil des Werkzeugs von Figur 2;
- Figur 4:: schematisch einen Ausschnitt zu Figur 1 mit einer Schraube zur Sicherung in Drehrichtung und in Axialrichtung; und
- Figur 5:: schematisch einen Schnitt durch einen Ausschnitt einer hohlen Wendel.

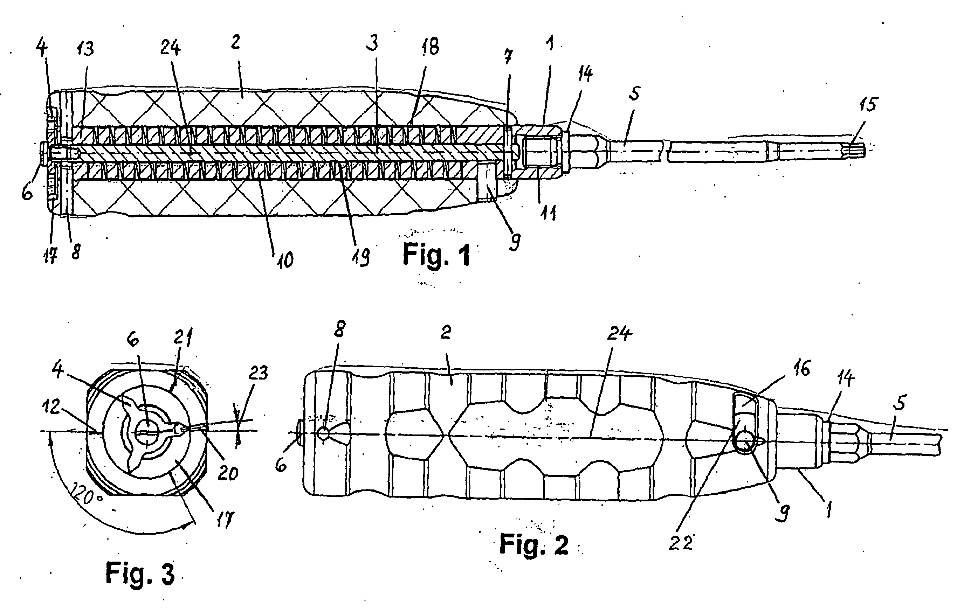

Mit den Figuren 1,2 und 3 ist eine erste Ausführungsform eines erfindungsgemässen Eindrehwerkzeuges gezeigt. Mit einem Längsgriff 2 ist ein Werkzeugteil 5, der in einem Sechskant 15 endet, indirekt verbunden. Als Zwischenglied fungiert eine Wendel 10, welche zum Werkzeugteil 5 hin in einem Kopfteil 1 endet, welches drehbar im Längsgriff 2 gelagert ist. Die Wendel 10 endet am anderen Ende in einem Fussteil 13, welches über zwei eingepresste Zylinderstifte 8 starr mit dem Längsgriff 2 verbunden ist.
Durch die Wendel 10, welche hohl ausgeführt ist, ist ein Stab 3 hindurchgeführt, der seinerseits im Kopfteil 1 mit einem eingepressten Zylinderstift 7 starr verankert ist. Der Stab 3 ist im Kopfteil 13 drehbar gelagert und ragt aus dem Kopfteil 13 heraus, damit ein Zeiger 4 mit einer Befestigungschraube 6 daran befestigt werden kann. Zwischen der Wendel 10 und dem Längsgriff 2 ist ein Spiel 18 vorhanden, welches verhindert, dass sich die Wendel verklemmt, wenn sie beim Auffedern ihren Durchmesser vergrössert. Zwischen der Wendel 10 und dem Stab 3 ist ein Spiel 19 vorgesehen, welches verhindert, dass sich die Wendel 10 beim Zusammenziehen auf dem Stab 3 festsetzt. Zur Aufnahme einer Axialkraft zwischen Kopfteil 1 und Längsgriff 2 ist ein Radialbolzen 9 im Kopfteil 1 eingepresst, welcher in einem quer zur Längsachse 24 verlaufenden Schlitz 22 beidseitig geführt ist und eine Drehbewegung bis zu einem Anschlag 16 erlaubt. Die aufzunehmenden Axialkräfte sind eher gering. Sie entstehen beim Ansetzen des Sechkants 15 an eine Schraube und beim Zusammenziehen und Öffnen der schraubenförmigen Wendel 10.
Der Werkzeugteil 5 ist auswechselbar mit dem Kopfteil 1 über ein Gewinde 11 verschraubt und sitzt mit einer Schulter 14 auf dem Kopfteil 1 auf. Auf diese Weise können verschiedene Werkzeuge für ein vorgegebenes Drehmoment eingesetzt werden.
In Figur 3 erkennt man, dass der Zeiger 4 dreifach mit einem Abstand von 120° ausgeführt ist, und dass Anzeige 12 für ein Sollmoment 21 ebenfalls dreifach mit einem Abstand von 120° ausgeführt ist, um ein Erreichen des Sollmoments aus verschiedenen Richtungen ablesen zu können. Einer der drei Zeiger ist mit einem "C" gekennzeichnet und steht in seiner Nulllage 20 einem markierten Toleranzwinkel 23 gegenüber, der eine zulässige Abweichung der Nulllage angibt.
Wie sich aus Figur 2 ergibt, steht der Radialbolzen 9 in der Nulllage fast am Ende des Schlitzes 22. Wird nun der Längsgriff nach rechts im Uhrzeigersinn gedreht, nachdem der Werkzeugteil 5 in Drehrichtung auf Widerstand gestossen ist, dann verdreht sich der Längsgriff mit seiner Anzeige 12 gegenüber dem Kopfteil 1 und damit auch gegenüber dem Zeiger 4. Das Drehmoment kann nun erhöht werden, bis das markierte Sollmoment 21 erreicht ist. Der Zeiger 4 ist in einer Aussparung 17 am Ende des Längsgriffs 2 eingelassen, damit er vor mechanischen Beschädigungen geschützt ist.

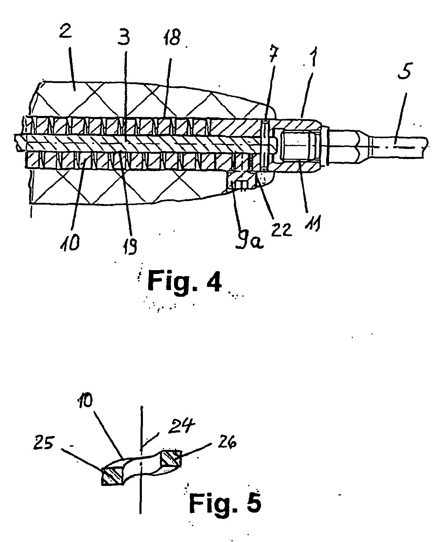

Ein weiteres Beispiel ist in Figur 4 gezeigt welches sich von Figur 1 nur dadurch unterscheidet, dass der eingepresste Radialbolzen 9 durch eine Schraube 9 a ersetzt wurde. Wenn man die eingepressten Zylinderstifte 8 (Figur 2) auch noch durch wieder lösbare Pass-Schrauben ersetzt, dann kann der Längsgriff 2 nach dem Entfernen von Pass-Schrauben und Schraube 9 a in Richtung der Achse 24 nach vorne abgezogen werden, was eine bessere Reinigung ermöglicht.

Figur 5 zeigt eine halbe Umdrehung der Wendel 10 mit den Querschnitten 25, 26. Bezüglich der Achse 24 tritt bei Torsion in jedem Querschnitt 25, 26 das gleiche Biegemoment auf. Bei einer durch die Praxis beschränkten Länge des Längsgriffes 2 entsteht durch die wesentlich grössere abgewickelte Länge der als Torsionsfeder benutzten hohlen Wendel für ein vorgegebenes Drehmoment ein Verdrehwinkel von beispielsweise 60°. Zum Beispiel kann die abgewickelte Länge der hohlen Wendel 10 dem 1,4-fachen der Länge des Längsgriffs 2 entsprechen. Ein Verdrehwinkel von dieser Grösse erlaubt es, ein vorgegebenes Drehmoment recht genau einzuhalten und auch eine relativ genaue Kontrolle der Nulllage vorzunehmen. Ein auf die Länge des Längsgriffes beschränkter Torsionsstab würde bei einer gleich beschränkten maximalen spezifischen Belastung nur eine minimale Verdrehung mit entsprechender Unsicherheit in der Ablesung zulassen.

### Bezugszeichenliste

- 1: Kopfteil
- 2: Längsgriff
- 3: Stab
- 4: Zeiger
- 5: Werkzeugteil
- 6: Befestigungsschraube
- 7: Zyl. - Stift
- 8: Zyl. - Stift
- 9: Radialbolzen
- 9 a: Schraube
- 10: Wendel
- 11: Gewinde
- 12: Anzeige
- 13: Fussteil
- 14: Schulter
- 15: Sechskant
- 16: Anschlag
- 17: Aussparung
- 18: Spiel
- 19: Spiel
- 20: Nulllage
- 21: Sollmoment
- 22: Schlitz
- 23: Toleranzwinkel
- 24: Achse
- 25, 26: Querschnitt

## Patentansprüche

1. Eindrehwerkzeug für Implantatschrauben mit einem Griff (2) und mit einem stabförmigen Werkzeug, wobei zur Momentübertragung eine elastische Wendel (10) vorgesehen ist, welche in ein eigentliches Werkzeugteil (5) mündet, wobei die Wendel (10) hohl ist und durch die Wendel (10) ein Stab (3) führt, um ausserhalb des Fussteils (13) mit einem Zeiger (4) die relative Verdrehung und das Drehmoment zwischen Werkzeugteil (5) und Griff (2) anzuzeigen, **dadurch gekennzeichnet, dass** der Griff ein Längsgriff ist, das Werkzeug im Längsgriff (2) verankert ist, die Wendel mit einem Fussteil (13) im Längsgriff (2) befestigt und mit einem Kopfteil (1) im Längsgriff (2) drehbar gelagert ist, der Stab im Kopfteil (1) fest verankert und im Fussteil (13) drehbar gelagert ist und die Wendel (10) mit Kopfteil (1) und Fussteil (13) einstückig ausgeführt ist.

2. Eindrehwerkzeug nach Anspruch 1, **dadurch gekennzeichnet, dass** zwischen Wendel (10) und Stab (3) ein Spiel (19) vorgesehen ist, das verhindert, dass sich die Wendel (10) beim Zusammenziehen auf dem Stab (3) festsetzt.

3. Eindrehwerkzeug nach einem der vorhergehenden Ansprüche, **dadurch gekennzeichnet, dass** der Kopfteil (1) mit einer in beiden Richtungen wirksamen Axialriegelung relativ zum Längsgriff versehen ist.

4. Eindrehwerkzeug nach einem der vorhergehenden Ansprüche, **dadurch gekennzeichnet, dass** der Griffteil einen Anschlag (16) für einen begrenzten Drehwinkel des Kopfteils (1) aufweist.

5. Eindrehwerkzeug nach Anspruch 4, **dadurch gekennzeichnet, dass** im Kopfteil (1) ein vorstehender Radialbolzen (9) verankert ist, welcher von einem Schlitz (22) im Längsgriff in seiner Schwenkung und in axialer Richtung begrenzt ist.

6. Eindrehwerkzeug nach Anspruch 4, **dadurch gekennzeichnet, dass** im Kopfteil (1) eine vorstehende Schraube (9a) verankert ist, welche von einem Schlitz (22) im Längsgriff in ihrer Schwenkung und in axialer Richtung begrenzt ist.

7. Eindrehwerkzeug nach einem der Ansprüche 5 oder 6, **dadurch gekennzeichnet, dass** der Schlitz für einen Drehwinkel zwischen 30° und 90° vorgesehen ist.

8. Eindrehwerkzeug nach einem der Ansprüche 1 bis 7, **dadurch gekennzeichnet, dass** der Werkzeugteil (5) mit dem Kopfteil (1) lösbar gekoppelt ist.

9. Eindrehwerkzeug nach einem der Ansprüche 1 bis 8, **dadurch gekennzeichnet, dass** der Längsgriff aus einem sterilisierbaren Kunststoff besteht.

10. Eindrehwerkzeug nach einem der Ansprüche 1 bis 9, **dadurch gekennzeichnet, dass** die Wendel (10) als Doppelwendel ausgeführt ist.

11. Eindrehwerkzeug nach einem der Ansprüche 1 bis 10, **dadurch gekennzeichnet, dass** in einer Null-Lage für die Verdrehung des Zeigers (4) ein Toleranzwinkel (23) am Griffteil markiert, ob die Null-Lage noch innerhalb einer zulässigen Grenze liegt.

12. Eindrehwerkzeug nach Anspruch 11, **dadurch gekennzeichnet, dass** der Toleranzwinkel mehr als plus/minus 2 ° beträgt.

13. Eindrehwerkzeug nach einem der vorhergehenden Ansprüche, **dadurch gekennzeichnet, dass** die Wendel (10) in ihrer abgewickelten Länge länger als der Längsgriff (2) ist.

14. Eindrehwerkzeug nach einem der vorhergehenden Ansprüche, **dadurch gekennzeichnet, dass** die Wendelgange einen Querschnitt aufweisen.

## Claims

1. A screw-driving tool for implant screws having a handle (2) and having a bar-shaped tool, wherein an elastic spiral (10) is provided for the torque transmission which opens into an actual tool part (5), with the spiral (10) being hollow and a bar (3) being guided through the spiral (10) in order to indicate the relative rotation and the torque between the tool part (5) and the handle (2) outside the foot part (13) by a pointer (4).
**characterized in that** the handle is a longitudinal handle, the tool is anchored in the longitudinal handle (2), the spiral has a foot part (13) secured in the longitudinal handle (2) and a head part (1) rotatably journalled in the longitudinal handle (2), the bar is fixedly anchored in the head part (1) and is rotatably journalled in the foot part (13) and the spiral (10) is made in one piece with the head part (1) and the foot part (13) .

2. A screw-driving tool in accordance with claim 1, **characterized in that** a clearance (19) is provided between the spiral (10) and the bar (19) which prevents the spiral (10) from seizing on the bar (3) on the pulling together.

3. A screw-driving tool in accordance with any one of the preceding claims, **characterized in that** the head part (1) is provided with an axial latch acting in both directions relative to the longitudinal handle.

4. A screw-driving tool in accordance with any one of the preceding claims, **characterized in that** the handle part has an abutment (16) for a limited angle of rotation of the head part (1).

5. A screw-driving tool in accordance with claim 4, **characterized in that** a projecting radial pin (9) is anchored in the head part (1) and is restricted in its pivotal movement and in the axial direction by a slot (22) in the longitudinal handle.

6. A screw-driving tool in accordance with claim 4, **characterized in that** a projecting screw (9a) is anchored in the head part (1) and is restricted in its pivotal movement and in the axial direction by a slot (22) in the longitudinal handle.

7. A screw-driving tool in accordance with any one of claims 5 or 6, **characterized in that** the slot is provided for an angle of rotation between 30° and 90°.

8. A screw-driving tool in accordance with any one of claims 1 to 7, **characterized in that** the tool part (5) is releasably coupled to the head part (1).

9. A screw-driving tool in accordance with any one of claims 1 to 8, **characterized in that** the longitudinal handle consists of a plastic which can be sterilized.

10. A screw-driving tool in accordance with any one of claims 1 to 9, **characterized in that** the spiral (10) is made as a double helix.

11. A screw-driving tool in accordance with any one of claims 1 to 10, **characterized in that**, in a zero position for the rotation of the pointer (4), a tolerance angle (23) at the handle part marks whether the zero position still lies within a permitted limit.

12. A screw-driving tool in accordance with claim 11, **characterized in that** the tolerance angle amounts to more than plus/minus 2°.

13. A screw-driving tool in accordance with any one of the preceding claims, **characterized in that** the spiral (10) in its unwound length is longer than the longitudinal handle (2).

14. A screw-driving tool in accordance with any one of the preceding claims, **characterized in that** the spiral pitches have a rectangular cross-section.

## Revendications

1. Outil de vissage pour vis d'implant, comportant un manche (2) et un outil en forme de tige, dans lequel une hélice (10) élastique est prévue pour transmettre le couple, laquelle débouche dans une partie d'outil (5) proprement dite, l'hélice (10) étant creuse et une tige (3) passant à travers l'hélice, pour indiquer par une aiguille (4) à l'extérieur de la partie de pied (13) la rotation relative et le couple entre la partie d'outil (5) et le manche (2), **caractérisé en ce que** le manche est un manche allongé, l'outil est ancré dans le manche allongé (2), l'hélice est fixée par une partie de pied (13) dans le manche allongé (2) et est montée rotative par une partie de tête (1) dans le manche allongé (2), la tige étant ancrée de manière solidaire dans la partie de tête (1) et montée rotative dans la partie de pied (13), et **en ce que** l'hélice (10) est réalisée d'un seul tenant avec la partie de tête (1) et la partie de pied (13).

2. Outil de vissage selon la revendication 1, **caractérisé en ce que** entre l'hélice (10) et la tige (3) est prévu un jeu (19) qui empêche que l'hélice se bloque lors du serrage sur la tige (3).

3. Outil de vissage selon l'une des revendications précédentes, **caractérisé en ce que** la partie de tête (1) est pourvue d'un verrouillage axial agissant dans les deux directions par rapport au manche allongé.

4. Outil de vissage selon l'une des revendications précédentes, **caractérisé en ce que** la partie de manche présente une butée (16) pour un angle de rotation limité de la partie de tête (1).

5. Outil de vissage selon la revendication 4, **caractérisé en ce que** dans la partie de tête (1) est ancré un boulon radial (9) en saillie qui est limité dans son pivotement et en direction axiale par une fente (22) dans le manche allongé.

6. Outil de vissage selon la revendication 4, **caractérisé en ce que** dans la partie de tête (1) est ancrée une vis (9a) en saillie qui est limitée dans son pivotement et en direction axiale par une fente (22) dans le manche allongé.

7. Outil de vissage selon l'une des revendications 5 ou 6, **caractérisé en ce que** la fente est prévue pour un angle de rotation entre 30° et 90°.

8. Outil de vissage selon l'une des revendications 1 à 7, **caractérisé en ce que** la partie d'outil (5) est accouplée à la partie de tête (1) de manière détachable.

9. Outil de vissage selon l'une des revendications 1 à 8, **caractérisé en ce que** le manche allongé est en une matière plastique susceptible d'être stérilisée.

10. Outil de vissage selon l'une des revendications 1 à 9, **caractérisé en ce que** l'hélice (10) est réalisée sous forme d'hélice double.

11. Outil de vissage selon l'une des revendications 1 à 10, **caractérisé en ce que** dans une position zéro pour la rotation de l'aiguille (4), un angle de tolérance (23) est marqué sur la partie de manche pour constater si la position zéro est encore située à l'intérieur d'une limite admissible.

12. Outil de vissage selon la revendication 11, **caractérisé en ce que** l'angle de tolérance est supérieur à plus/moins 2°.

13. Outil de vissage selon l'une des revendications précédentes, **caractérisé en ce que** l'hélice (10) est, dans sa longueur déroulée, plus grande que le manche allongé (2).

14. Outil de vissage selon la revendication 1, **caractérisé en ce que** les spires de l'hélice présentent une section transversale rectangulaire.
